# EUROPEAN PATENT APPLICATION

(11) **EP 4 406 409 A1**
(43) Date of publication of application: **31.07.2024**
(21) Application number: 21957585.9
(22) Date of filing: 20.09.2021
(51) Int. Cl.: A01K 67/033

(54) **REARING DEVICE**

(71) Applicant: JTEKT Corporation, Kariya-shi, Aichi-ken, 448-8652 (JP); Tokushima University, Tokushima-shi, Tokushima 770-8501 (JP)
(72) Inventor: TAKAZATO Akihiro, Kariya-shi, Aichi 448-8652 (JP); NAKAMURA Teruhiro, Kariya-shi, Aichi 448-8652 (JP); KAWAI Shigekazu, Kariya-shi, Aichi 448-8652 (JP); MURATA Yasuhiro, Kariya-shi, Aichi 448-8652 (JP); MIURA Nozomu, Kariya-shi, Aichi 448-8652 (JP); WATANABE Takahito, Tokushima-shi, Tokushima 770-8501 (JP); MITO Taro, Tokushima-shi, Tokushima 770-8501 (JP)
(74) Representative: TBK
(86) International application number: PCT/JP2021/034441
(87) International publication number: WO 2023/042404

(57) **Abstract**

A breeding device (1) includes: a body frame (10) forming a breeding space (11) for organisms; a plurality of breeding units (20) housed in the breeding space (11) of the body frame (10); a plurality of perch members (22) arranged in each of the plurality of breeding units (20) with a clearance between the perch members; and a separating member (32) including a plurality of comb teeth (34) arranged in an arrangement direction (Y) of the plurality of perch members (22). The separating member (32) is used for all of the plurality of breeding units (20), and is configured such that the plurality of comb teeth (34) is inserted into a plurality of the clearances (23) between the plurality of perch members (22) by moving the separating member (32) relative to each of the plurality of breeding units (20).

## Description

### TECHNICAL FIELD

The present disclosure relates to breeding devices.

### BACKGROUND ART

Patent Document 1 describes a cricket breeding device. In this breeding device, a container in which water-soaked absorbent cotton is placed is provided in a breeding case in order to water crickets. Spawning, hatching, and breeding of crickets are all carried out in this breeding case.

### Related Art Documents

### Patent Documents

Patent Document 1: Japanese Unexamined Patent Application Publication No. H10-191834 (JP H10-191834 A)

### SUMMARY OF THE INVENTION

In the above breeding device, crumpled newspapers can be used to create a hiding environment for crickets. When collecting crickets, a worker needs to unfold the crumpled newspapers one by one and separate the crickets from the unfolded newspapers. Therefore, the work of collecting crickets may take a lot of time and effort.

An object of the present disclosure is to provide a technique that is effective in efficiently collecting bred organisms in a short time in a breeding device for breeding organisms such as insects.

One aspect of the present disclosure is a breeding device for organisms. The breeding device includes: a body frame forming a breeding space for the organisms; a plurality of breeding units housed in the breeding space of the body frame; a plurality of perch members arranged in each of the plurality of breeding units with a clearance between the perch members; and a separating member including a plurality of comb teeth arranged in an arrangement direction of the plurality of perch members. The separating member is used for all of the plurality of breeding units, and is configured in such a manner that the plurality of comb teeth is inserted into a plurality of the clearances between the plurality of perch members by moving the separating member relative to each of the plurality of breeding units.

### Effects of the Invention

The breeding device of the above aspect includes the body frame, the plurality of breeding units, the plurality of perch members, and the separating member. In this breeding device, the plurality of breeding units is housed in the breeding space of the body frame. The plurality of perch members arranged with a clearance therebetween is provided in each of the plurality of breeding units. The separating member includes the plurality of comb teeth arranged in the arrangement direction of the plurality of perch members.

The separating member is used for all of the plurality of breeding units, and is used to separate the organisms perching on the plurality of perch members of each breeding unit from the plurality of perch members. For this purpose, the separating member is moved relative to each of the plurality of breeding units after the breeding during which the organisms have grown. The plurality of comb teeth is thus inserted into the plurality of clearances between the plurality of perch members of each breeding unit. By causing each comb tooth of the separating member to interfere with the organisms located in the clearance between the two perch members facing each other, the organisms can be easily separated from the plurality of perch members of each breeding unit. At this time, one separating member need only be moved relative to each of the plurality of breeding units. This can reduce the time it takes to separate and collect the organisms from the plurality of perch members of each breeding unit, compared to a structure in which a separating member is prepared for each of the plurality of breeding units and the separate separating members are moved relative to the breeding units.

According to the above aspect, it is possible to provide a technique that is effective in efficiently collecting bred organisms in a short time in a breeding device for breeding organisms such as insects.

The signs in parentheses in the claims indicate the correspondence with specific means described in the embodiments described below, and are not intended to limit the technical scope of the present disclosure.

### BRIEF DESCRIPTION OF THE DRAWINGS

The foregoing and other objects, features, and advantages of the present disclosure will become more apparent from the following detailed description when taken in conjunction with the accompanying drawings.
[FIG. 1] FIG. 1 is a perspective view of a breeding device of a first embodiment with a collection device connected to a body frame.
[FIG. 2] FIG. 2 is a perspective view of the breeding device of the first embodiment with the collection device separated from the body frame.
[FIG. 3] FIG. 3 is a perspective view of a breading unit.
[FIG. 4] FIG. 4 is a front view showing the state in which a first breeding unit and a second breeding unit are supported by support portions of the body frame.
[FIG. 5] FIG. 5 is a front view showing the state in which the first breeding unit and the second breeding unit are supported by support portions of the collection device.
[FIG. 6] FIG. 6 is a perspective view of the collection device in FIG. 2 as viewed from the body frame side.
[FIG. 7] FIG. 7 is a front view showing the states before and after comb teeth of a separating member are inserted into clearances between perch members of the first breeding unit.
[FIG. 8] FIG. 8 is a side view showing the configuration of a belt conveyor under the first breeding unit.
[FIG. 9] FIG. 9 is a side view showing the state before the comb teeth of the separating member are inserted into the clearances between the perch members of the first breeding unit.
[FIG. 10] FIG. 10 is a side view showing the state immediately after the comb teeth of the separating member are inserted into the clearances between the perch members of the first breeding unit.
[FIG. 11] FIG. 11 is a partial enlarged view of the perch members of the first breeding unit.
[FIG. 12] FIG. 12 is a side view showing the state in which the comb teeth of the separating member are further inserted into the clearances between the perch members of the first breeding unit from the state shown in FIG. 10.
[FIG. 13] FIG. 13 is a side view showing the state immediately after the comb teeth of the separating member pass through the clearances between the perch members of the first breeding unit.
[FIG. 14] FIG. 14 is a side view of a separating member according to a modification.
[FIG. 15] FIG. 15 is a side view of a separating member according to a modification.
[FIG. 16] FIG. 16 is a partial enlarged view of a comb tooth of a separating member according to a modification.
[FIG. 17] FIG. 17 is a partial enlarged view of a comb tooth of a separating member according to a modification.
[FIG. 18] FIG. 18 is a sectional view taken along line XVIII-VIII in FIG. 17.
[FIG. 19] FIG. 19 is a partial enlarged view of a comb tooth of a separating member according to a modification.
[FIG. 20] FIG. 20 is a side view of a breeding device of a second embodiment.
[FIG. 21] FIG. 21 is a side view of a breeding device of a third embodiment with a separating member located at a raised position.
[FIG. 22] FIG. 22 is a side view of the breeding device of the third embodiment with the separating member located at a lowered position.

### MODES FOR CARRYING OUT THE INVENTION

Hereinafter, a specific structure of a breeding device that is an embodiment of the above aspect will be described with reference to the drawings.

In the specification and drawings, arrow X indicates a first direction that is a vertical direction of the breeding device, arrow Y indicates a second direction that is a horizontal direction of the breeding device, and arrow Z indicates a third direction that is a horizontal direction of the breeding device and that is perpendicular to the second direction, unless otherwise specified.

### (First Embodiment)

### 1. Breeding Targets

Organisms to be bred in the breeding device, namely breeding targets, are small organisms such as arthropods. The breeding targets are, for example, organisms to be used for food, feed, research, etc. For example, the breeding targets can be insects such as crickets, locusts, and grasshoppers. In particular, the organisms that are the breeding targets are suitably arthropods with incomplete metamorphosis in which they change directly from nymph to adult, more preferably arthropods nymphs undergoing incomplete metamorphosis. In this example, nymphs of crickets and locusts in the order Orthoptera among insects will be described as suitable examples of organisms that are the breeding targets. However, the breeding targets may be adults (emerged arthropods with incomplete metamorphose). The breeding device may be used to breed organisms from eggs or to breed organisms after hatching.

### 2. Basic Configuration of Breeding Device

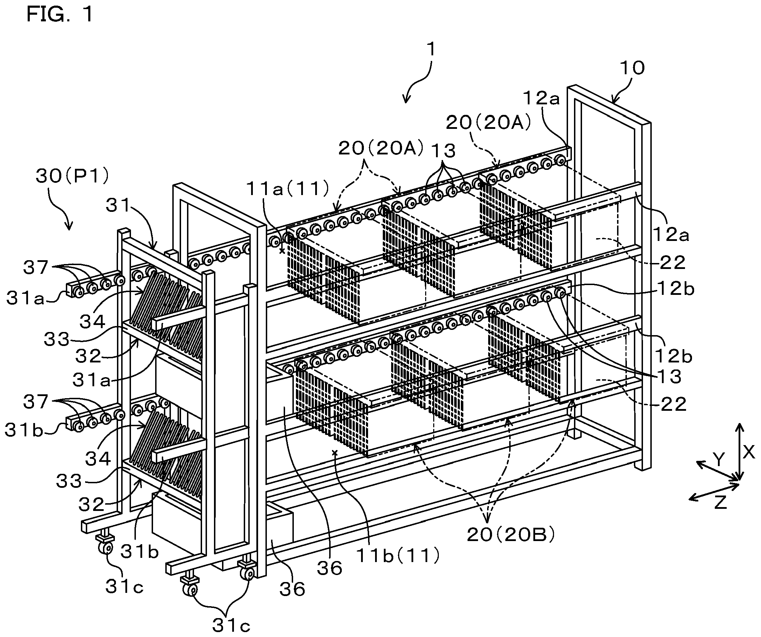

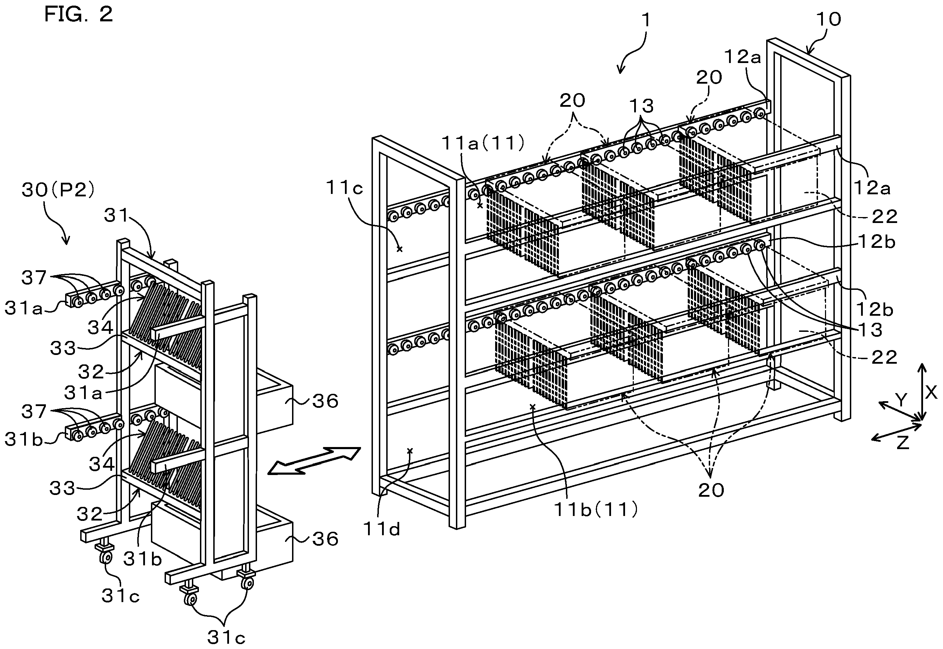

As shown in FIGS. 1 and 2, a breeding device 1 of the first embodiment is used to breed organisms C that are the breeding targets. In FIG. 1, crickets that are a type of insect are exemplified as the organisms C. The breeding device 1 includes, as its basic configuration, a body frame 10, a plurality of breeding units 20, and a collection device 30.

The body frame 10 is placed on an installation surface for the breeding device 1. The body frame 10 is configured to form a breeding space 11 for the organisms C. Although not shown in FIG. 1 for convenience, the outer periphery of the body frame 10 is covered by a wall portion that defines the breeding space 11. The breeding space 11 is therefore a closed space that is always closed during the breeding period, and this closed space is set as appropriate to an environment suitable for the growth of the organisms C.

### 3. Internal Structure of Body Frame

In this example, two breeding spaces 11 are formed in two tiers in the first direction X, namely upper and lower tiers, inside the body frame 10. That is, a first breeding space 11a located in the upper tier in the first direction X and a second breeding space 11b located in the lower tier in the first direction X are formed as the breeding spaces 11. The first breeding space 11a and the second breeding space 11b are spaces of the same dimensions.

As shown by long dashed double-short dashed lines in FIGS. 1 and 2, the plurality of breeding units 20 is housed in the breeding spaces 11 inside the body frame 10. The plurality of breeding units 20 include three first breeding units 20A and three second breeding units 20B. The first breeding unit 20A and the second breeding unit 20B have the same shape.

The three first breeding units 20A are housed side by side in the third direction Z in the first breeding space 11a. The three second breeding units 20B are housed side by side in the third direction Z in the second breeding space 11b. The number of first breeding units 20A and the number of second breeding units 20B are not particularly limited, and can be changed as appropriate when necessary.

The body frame 10 includes a plurality of support portions 12a, 12b extending in the third direction Z. Two support portions 12a are components for supporting the first breeding units 20A so as to allow the first breeding units 20A to slide in the third direction Z, and extend spaced apart from each other in the second direction Y and parallel to the third direction Z. Two support portions 12b are components for supporting the second breeding units 20B so as to allow the second breeding units 20B to slide in the third direction Z, and extend spaced apart from each other in the second direction Y and parallel to the third direction Z.

A plurality of support rollers 13 is arranged along the third direction Z on each of the inner surfaces in the second direction Y of the support portion 12a and the support portion 12b. The support roller 13 is a rotatable member such as a roller or a bearing, and is configured to rotate upon receiving input in the third direction Z.

Although not shown in FIGS. 1 and 2, a belt conveyor 40 described later is installed in each of an area under the first breeding units 20A and an area under the second breeding units 20B inside the body frame 10.

### 4. Structure of Breeding Unit

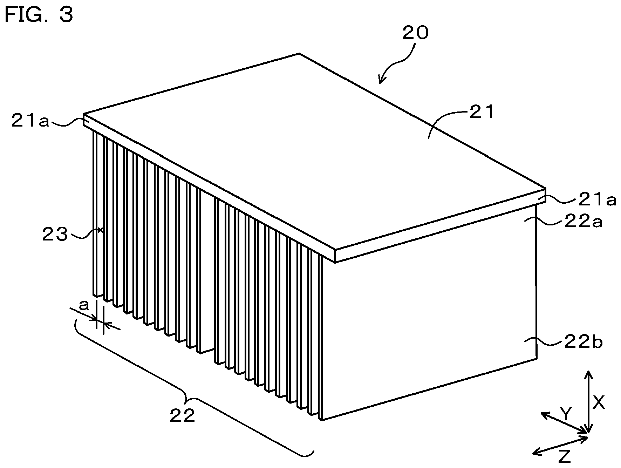

As shown in FIG. 3, each of the plurality of breeding units 20 includes a plurality of perch members 22 and a holding portion 21 that holds the plurality of perch members 22. The plurality of perch members 22 has the same shape and is configured so as to hang down from the lower surface of the holding portion 21 serving as a top plate. The plurality of perch members 22 extends downward to their lower ends 22b, and their base ends 22a are the ends closer to the holding portion 21. The plurality of perch members 22 is arranged in the second direction Y with clearances 23 indicated by a dimension a in FIG. 3 therebetween. That is, the plurality of perch members 22 is arranged at substantially equal intervals in the second direction Y

The perch member 22 is a plate-like member extending on a plane defined by the first direction X and the third direction Z, and its thickness direction is the second direction Y. The perch member 22 is configured to allow the organisms C that are the breeding targets to perch on its surface. The perch member 22 may be formed in the shape of a flat plate or may be formed in a corrugated shape. In this example, the perch member 22 is formed in the shape of a flat plate.

The material of the perch member 22 is not particularly limited, but for example, metal such as iron or aluminum, resin, wood, or paper can be used. It is preferable that the perch member 22 have a mesh structure having a large number of through holes formed all over its entire surface so as to serve as footholds for the organisms C. For example, the perch member 22 can be made of punched metal or wire mesh.

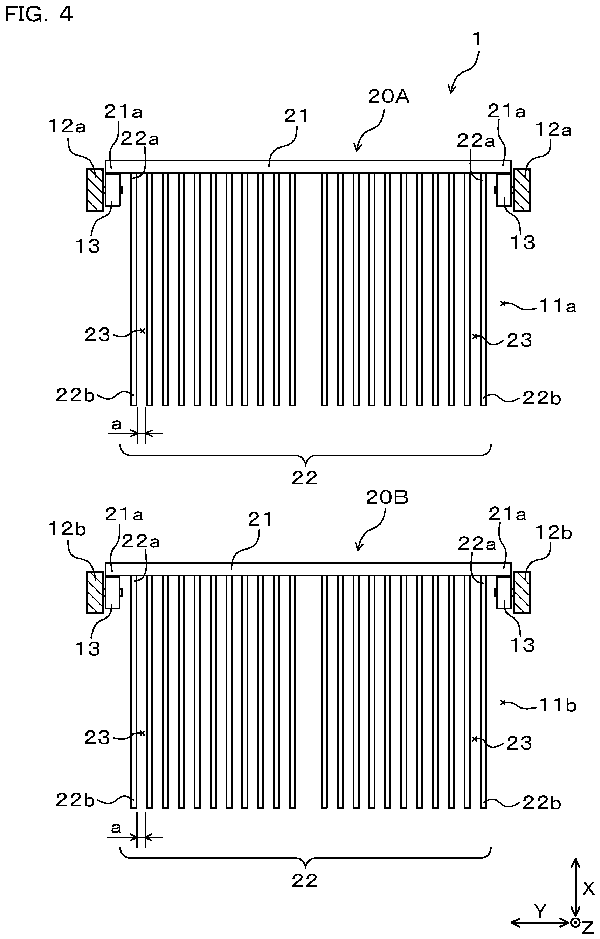

As shown in FIG. 4, the first breeding unit 20A is housed in the first breeding space 11a inside the body frame 10, with both ends 21a in the second direction Y of the holding portion 21 being supported by the plurality of support rollers 13 of the support portions 12a from below. Like the first breeding unit 20A, the second breeding unit 20B is housed in the second breeding space 11b inside the body frame 10, with both ends 21a in the second direction Y of the holding portion 21 being supported by the plurality of support rollers 13 of the support portions 12b from below.

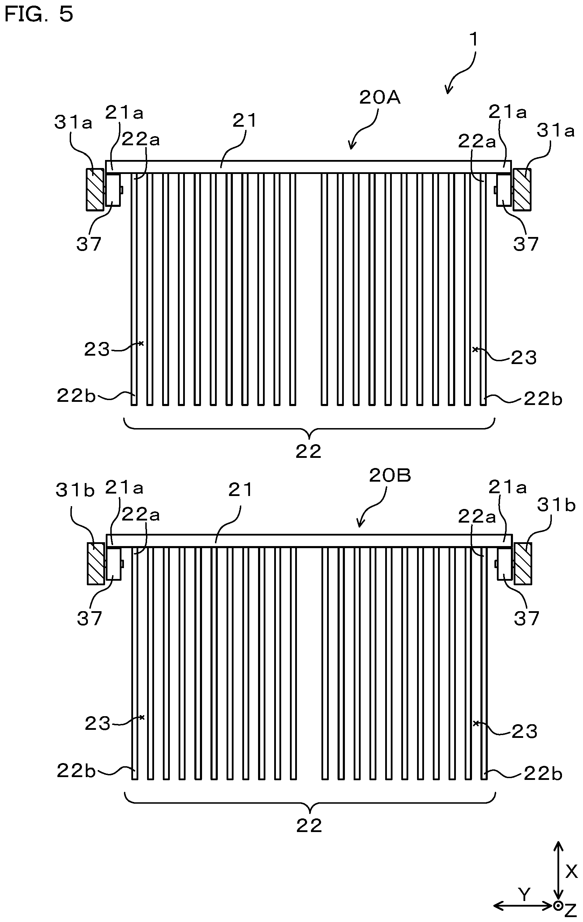

As shown in FIG. 5, when the first breeding unit 20A moves from the body frame 10 side to the collection device 30 side, both ends 21a in the second direction Y of the holding portion 21 are supported by a plurality of support rollers 37 of support portions 31a from below. Like the first breeding unit 20A, when the second breeding unit 20B moves from the body frame 10 side to the collection device 30 side, both ends 21a in the second direction Y of the holding portion 21 are supported by a plurality of support rollers 37 of support portions 31b from below. The support roller 37 has the same structure as the support roller 13.

### 5. Structure of Collection Device

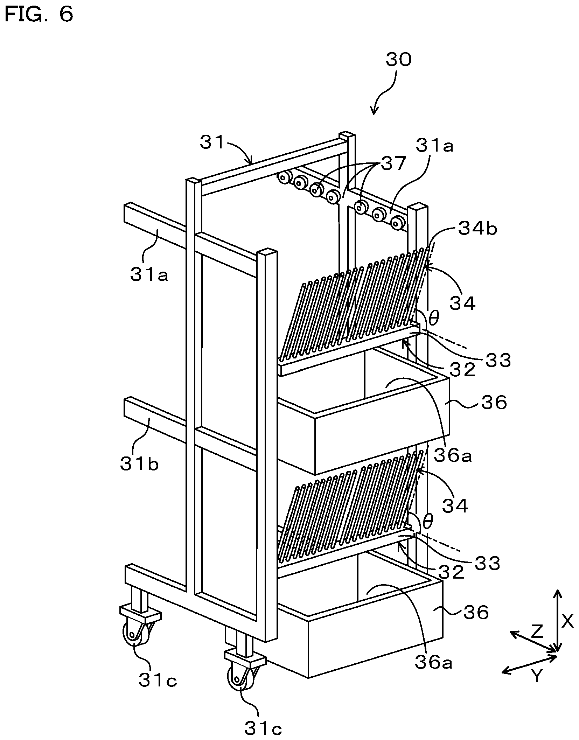

The collection device 30 is a device separate from the body frame 10, and is used when collecting the organisms C after the end of the breeding period. The collection device 30 is connected to the body frame 10 when collecting the organisms C (see FIG. 1), and is separated from the body frame 10 when breeding the organisms C (see FIG. 2). As shown in FIG. 6, a body portion 31 of the collection device 30 is provided with two separating members 32 and two cases 36.

The separating member 32 is a member for separating the organisms C from the perch members 22, described below, of each breeding unit 20 by interfering with the organisms C perching on the perch members 22. The upper separating member 32 is provided so as to face an opening 11c (see FIG. 2) in the third direction Z of the first breeding space 11a. The lower separating member 32 is provided so as to face an opening 11d (see FIG. 2) in the third direction Z of the second breeding space 11b.

The case 36 is a member for receiving and collecting the organisms C separated from the perch members 22 by the separating member 32 from below. The case 36 is a member in the shape of a bottomed box, and has a collection space 36a with open top. The upper case 36 is provided below the upper separating member 32. The lower case 36 is provided below the lower separating member 32.

The body portion 31 includes a plurality of support portions 31a, 31b extending in the third direction Z. Like the support portions 12a, two support portions 31a are components for supporting the plurality of first breeding units 20A so as to allow the first breeding units 20A to slide in the third direction Z, and extend spaced apart from each other in the second direction Y and parallel to the third direction Z. Two support portions 31b are components for supporting the plurality of second breeding units 20B so as to allow the second breeding units 20B to slide in the third direction Z, and like the support portions 12b, extend spaced apart from each other in the second direction Y and parallel to the third direction Z.

The plurality of support rollers 37 that is the same as the support rollers 13 is arranged along the third direction Z on each of the inner surfaces in the second direction Y of the support portion 31a and the support portion 31b. Like the support roller 13, the support roller 37 is configured to rotate upon receiving input in the third direction Z.

A plurality of casters 31c that allows the collection device 30 to travel is provided at the bottom of the body portion 31. This allows the collection device 30 to move with respect to the body frame 10 when a worker manually operates the collection device 30.

When the collection device 30 is in a connected position P1 (see FIG. 1) where the collection device 30 is connected to the body frame 10, the support portions 31a are located on lines extended from the support portions 12a of the body frame 10. Similarly, the support portions 31b are located on lines extended from the support portions 12b of the body frame 10. Therefore, when the collection device 30 is in the connected position P1, the first breeding units 20A and the second breeding units 20B can be caused to slide as desired in the third direction Z between the body frame 10 having the support rollers 13 and the collection device 30 having the support rollers 37.

The collection device 30 is not used during the breeding period of the organisms C. Therefore, the collection device 30 is connected to the body frame 10 only when collecting the organisms C. In the period other than the collection period, the collection device 30 can be separated from the body frame 10 and moved from the connected position P1 to a standby position P2 (see FIG. 2).

### 6. Structure of Separating Member

As shown in FIG. 6, the separating member 32 includes a support portion 33 fixed to the body portion 31 and extending in the second direction Y, and a plurality of comb teeth 34. The support portion 33 supports the plurality of comb teeth 34, and the plurality of comb teeth 34 extends from the support portion 33 side to their tip ends 34b. The material of the separating member 32 is not particularly limited, but for example, metal such as iron or aluminum, resin, or wood can be used.

The plurality of comb teeth 34 is rod-shaped portions having the same shape. The cross-sectional shape of each comb tooth 34 is not particularly limited, and may be any suitable shape such as a circle, an ellipse, a rectangle, or a polygon.

The plurality of comb teeth 34 is tilted at a certain tilt angle θ from the support portion 33 side to the tip ends 34b with respect to the third direction Z that is the direction in which the separating member 32 moves relative to the plurality of breeding units 20. All of the plurality of comb teeth 34 are tilted toward the body frame 10. The tilt angle θ can be set as appropriate within the range of 30° to 90°.

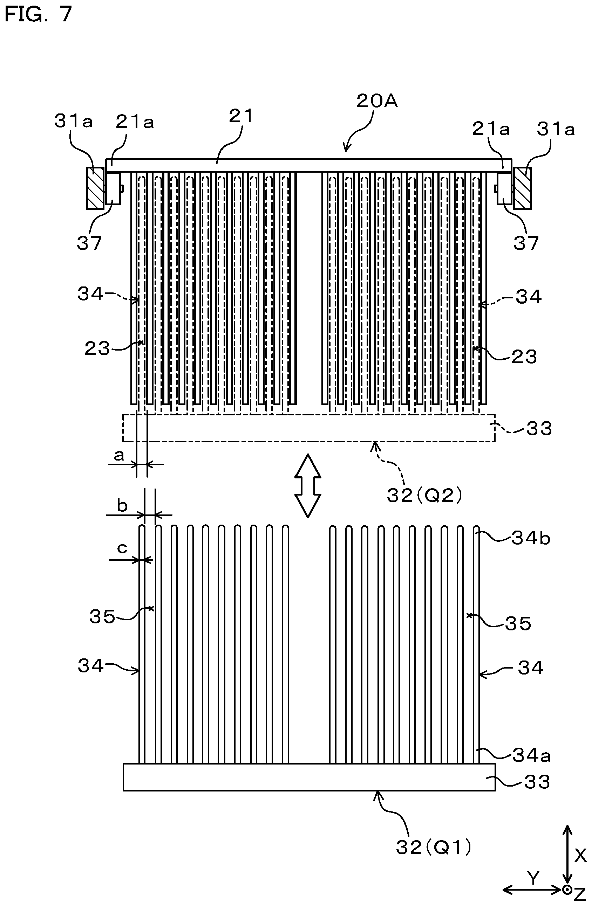

As shown in FIG. 7, the plurality of comb teeth 34 of the separating member 32 is arranged at equal intervals in the same direction as the second direction Y that is the direction of arrangement of the plurality of perch members 22, with clearances 35 (shown by a dimension a in FIG. 7) therebetween. That is, the plurality of comb teeth 34 of the separating member 32 is arranged parallel to each other in the second direction Y.

The separating member 32 is configured so that the dimension b in the second direction Y of the clearance 35 and the dimension c in the second direction Y that is the width direction of the comb tooth 34 have a correlation as given by a = b > c with the dimension a in the second direction Y of the clearance 23. Therefore, the separating member 32 can be in either an uninserted state Q1 in which the comb teeth 34 are separated from the clearances 23 or an inserted state Q2 in which part or all of the comb teeth 34 are inserted in the clearances 23.

Although FIG. 7 shows only the relationship of the separating member 32 with the perch members 22 of the first breeding unit 20A, the same relationship applies to the second breeding unit 20B. Therefore, the relationship of the separating member 32 with the perch members 22 of the second breeding unit 20B is not shown in the figure.

This example illustrates the case where the plurality of comb teeth 34 of the separating member 32 is arranged at equal intervals. However, the separating member 32 may be configured so that the plurality of comb teeth 34 is arranged at unequal intervals, as necessary.

### 7. Structure of Belt Conveyor

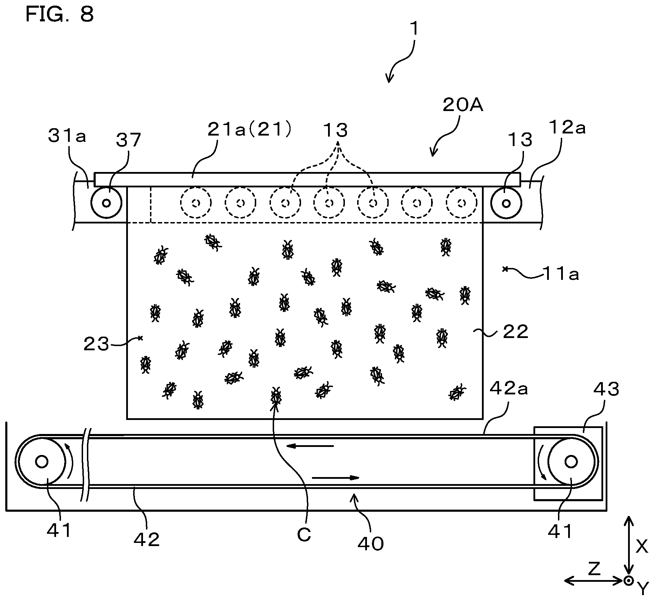

The belt conveyor 40 is installed in each of the first breeding space 11a and the second breeding space 11b. FIG. 8 shows the belt conveyor 40 installed in the first breeding space 11a. The belt conveyor 40 includes a pair of rollers 41, 41, an endless belt 42 wound around the pair of rollers 41, 41, and a motor 43 that drives one of the rollers 41.

In the belt conveyor 40, the motor 43 drives one of the rollers 41, so that a conveying surface 42a of the belt 42 moves continuously. At this time, the conveying surface 42a of the belt 42 forms the bottom surface of the breeding space 11. The conveying direction of the belt conveyor 40 means the moving direction of the belt 42. The motor 43 and the rollers 41, 41 may be rotatable in only one direction or may be rotatable in both directions. That is, when the motor 43 is rotatable in only one direction, the conveying direction of the belt conveyor 40 is a direction in which the conveying surface 42a of the belt 42 moves from right to left in FIG. 8. When the motor 43 is rotatable in both directions, the conveying direction of the belt conveyor 40 is both a first conveying direction in which the conveying surface 42a of the belt 42 moves from right to left in FIG. 8 and a second conveying direction in which the conveying surface 42a of the belt 42 moves from left to right in FIG. 8.

The belt conveyor 40 conveys at least one of the following: the organisms C that are the breeding targets, shells of the organisms C, food, water, and dust. For example, the belt conveyor 40 can be used to carry the organisms C in the early stage of growth or eggs of the organisms C from the outside into the first breeding space 11a. The organisms C bred in the first breeding space 11a need food and water. Therefore, the belt conveyor 40 can be used to supply food and water from the outside into the first breeding space 11a. Shells, feces, and food leftovers of the organisms, dead organisms C, etc. accumulate at the bottom of the first breeding space 11a during breeding of the organisms C. Therefore, the belt conveyor 40 can be used to carry them from the bottom of the first breeding space 11a to the outside. Although not particularly shown in the figure, the belt conveyor 40 can be used in the same manner for the second breeding space 11b.

### 8. Operation of Breeding Device

The operation of the breeding device 1 will be described with reference to FIGS. 1 and 9 to 13.

In this example, the first breeding unit 20A in the upper tier and the second breeding unit 20B in the lower tier operate in the same manner. Therefore, for convenience, only the operation of the first breeding unit 20A will be described below, and description of the operation of the second breeding unit 20B will be omitted.

As shown in FIG. 1, the organisms C are bred in the first breeding space 11a and the second breeding space 11b inside the body frame 10 during the breeding period. When the breeding period of the organisms C ends, the collection work is performed. In the collection work, the collection device 30 is first set to the connected position P1 where the collection device 30 is connected to the body frame 10.

The collection device 30 is disposed such that the upper separating member 32 faces the first breeding space 11a in the third direction Z and the two support portions 31a extends continuously with and are connected to the two support portions 12a of the body frame 10. This connection to the body frame 10 allows the collection device 30 to guide the plurality of first breeding units 20A such that they can continuously move from the support portions 12a of the body frame 10 to the support portions 31a of the collection device 30.

Subsequently, the three first breeding units 20A housed in the first breeding space 11a are sequentially moved toward the collection device 30 in order from the one closest to the collection device 30. The breeding units 20A, 20B do not have self-propelled means. Therefore, at this time, the worker can move the breeding units 20A, 20B in a moving direction Z1 by directly holding the breeding units 20A, 20B with fingers and moving them manually.

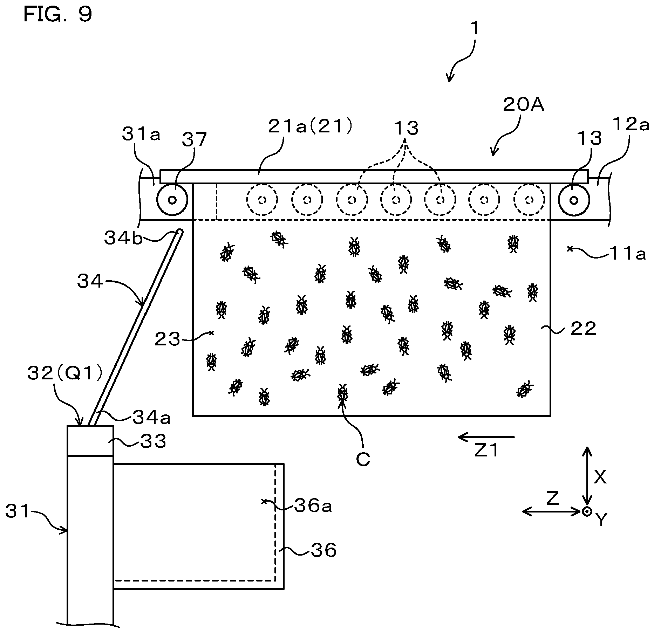

As shown in FIG. 9, the first breeding unit 20A to be moved first is moved in the moving direction Z1 with the both ends 21a of its holding portion 21 supported by the plurality of support rollers 13 of the support portions 12a from below. In the following description, the operation of the first breeding unit 20A at this time is also simply referred to as "sliding." At this time, the support portions 12a of the body frame 10 serve as first support portions that support the plurality of first breeding units 20A such that the plurality of first breeding units 20A are movable in the third direction Z with respect to the separating member 32 in a stationary state. When this first breeding unit 20A is slid in the moving direction Z1, the remaining two first breeding units 20A are in a standby state in the first breeding space 11a.

As the first breeding unit 20A slides, the separating member 32 moves relative to the first breeding unit 20A in the third direction Z. As a result, the front sides in the moving direction Z1 of both ends 21a of the holding portion 21 slide onto the support rollers 37 of the support portion 31a, so that both ends 21a are supported from below. At this time, the support portions 31a serve as second support portions that support the plurality of first breeding units 20A such that the plurality of first breeding units 20A are movable in the third direction Z.

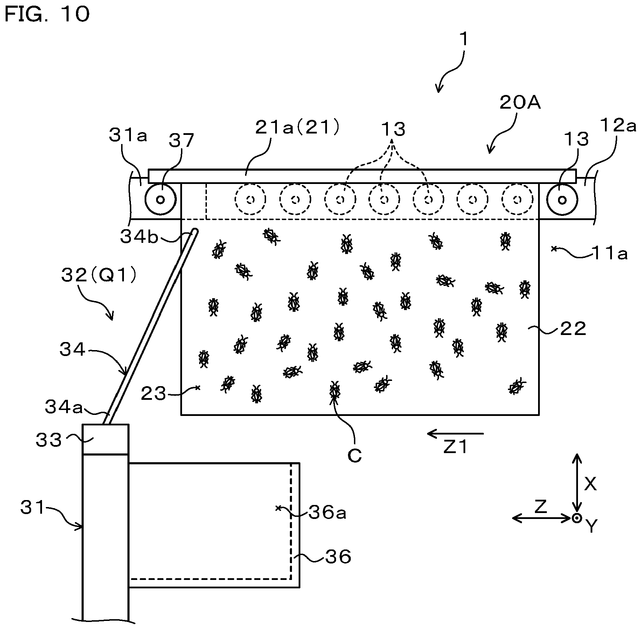

As shown in FIG. 10, when the first breeding unit 20A is further slid in the moving direction Z1 from the state shown in FIG. 9, the plurality of comb teeth 34 of the separating member 32 is inserted into the plurality of clearances 23 between the plurality of perch members 22 with the first breeding unit 20A supported by the support portions 31a. The separating member 32 thus switches from the uninserted state Q1 to the inserted state Q2.

At this time, due to the tilted structure of the plurality of comb teeth 34, their tip ends 34b far from the support portion 33 are first inserted into the clearances 23 at the base ends 22a of the perch members 22. Thereafter, the comb teeth 34 are further inserted into the clearances 23, so that the tilted portions from the tip ends 34b to base ends 34a act to correct deflection in the second direction Y of the separating member 32. The base ends 34a of the comb teeth 34 that are the ends closer to the support portion 33 are then eventually inserted into the clearances 23.

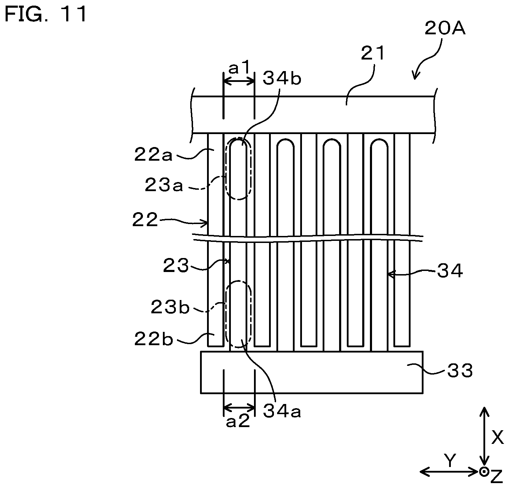

As shown in FIG. 11, the tip ends 34b of the comb teeth 34 are inserted into first regions 23a of the clearances 23 between the perch members 22 that are the regions close to the holding portion 21, but are not inserted into second regions 23b of the clearances 23 between the perch members 22 that are the regions far from the holding portion 21. On the other hand, the base ends 34a of the comb teeth 34 are inserted into the second regions 23b of the clearances 23 between the perch members 22, but are not inserted into the first regions 23a.

The first region 23a is a region defined by the wall surfaces of the base ends 22a of the root portions of the perch members 22 that are located near the holding portion 21. Therefore, the base end 22a of the perch member 22 is less likely to deflect and deform in the second direction Y, and the clearance dimension a1 in the second direction Y of the first region 23a approximately does not change.

On the other hand, the second region 23b is a region defined by the wall surfaces of the lower ends 22b of the perch members 22 that are far from the holding portion 21. Therefore, the lower end 22b of the perch member 22 tends to deflect and deform in the second direction Y, and the clearance dimension a2 in the second direction Y of the second region 23b may change with this deflection and deformation. For example, when the clearance dimension a2 of the second region 23b is smaller than the clearance dimension a1 of the first region 23a, a problem may occur that the operation of inserting the comb teeth 34 into the second regions 23b is hindered.

Therefore, in this example, a structure is used in which the tip ends 34b of the comb teeth 34 are inserted into the first regions 23a of the clearances 23 between the perch members 22 that are less susceptible to the deflection and deformation of the perch members 22. This allows smooth insertion of the comb teeth 34 of the separating member 32 into the clearances 23 between the perch members 22.

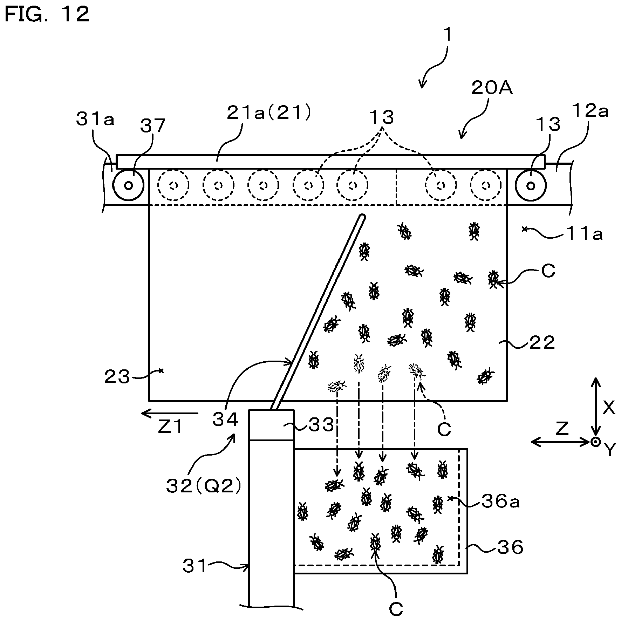

As shown in FIG. 12, when the first breeding unit 20A is further slid in the moving direction Z1 from the state shown in FIG. 11, the comb teeth 34 of the separating member 32 interfere with the organisms C perching on part of each perch member 22, so that these organisms C can be physically separated from the perch members 22. Part of the organisms C separated from the perch members 22 is collected into the collection space 36a of the case 36 located below the comb teeth 34 as, for example, they fall due to their own weight.

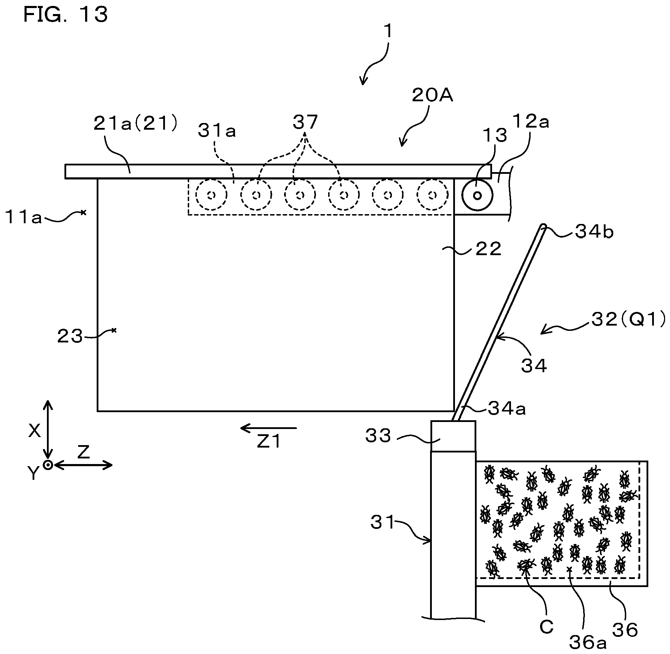

As shown in FIG. 13, the first breeding unit 20A is further slid in the moving direction Z1 from the state shown in FIG. 12 until the perch members 22 pass the separating member 32. The first breeding unit 20A thus passes through the separating member 32. At this time, the comb teeth 34 of the separating member 32 interfere with all of the remaining organisms C perching on the perch members 22, so that these organisms C can be physically separated from the perch members 22. The remaining organisms C thus separated from the perch members 22 are collected into the collection space 36a of the case 36 located below the comb teeth 34 as, for example, they fall due to their own weight. It is preferable to set the dimension c in the second direction Y of the comb teeth 34 so that the separating member 32 can interfere with the organisms C present in the clearances 23 and reliably scrape off these organisms C.

The tip ends 34b of the plurality of comb teeth 34 are caused to leave the clearances 23 before the base ends 34a thereof, and the base ends 34a are then caused to leave the clearances 23. When the plurality of comb teeth 34 is thus caused to leave the plurality of clearances 23 between the plurality of perch members 22, the separating member 32 switches from the inserted state Q2 to the uninserted state Q1 again.

After the first breeding unit 20A is removed from the collection device 30 in the moving direction Z1, the organisms C collected in the collection space 36a of the case 36 are collected. Thereafter, the first breeding unit 20A is cleaned as appropriate for reuse.

The operation described with reference to FIGS. 10 to 13 is also used for the remaining first breeding units 20A that are standing by in the first breeding space 11a. That is, the remaining first breeding units 20A are sequentially slid toward the separating member 32 and sequentially passed through the separating member 32. The organisms C perching on the perch members 22 of the remaining first breeding units 20A can thus be separated from the perch members 22 using the comb teeth 34 of the separating member 32, and the separated organisms C can be collected into the collection space 36a of the case 36.

As described above, in this example, the separating member 32 is used for all of the plurality of first breeding units 20A. By moving the separating member 32 relative to each of the first breeding units 20A, the plurality of comb teeth 34 is inserted into the plurality of clearances 23 between the plurality of perch members 22, so that the organisms C can be collected from the clearances 23. The work of collecting the organisms C is therefore a simple work of merely sliding the first breeding units 20A in the moving direction Z1.

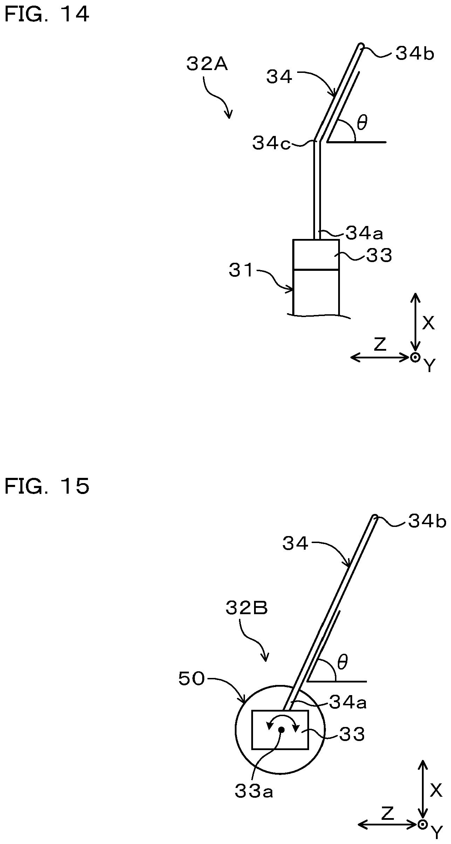

Next, modifications of the separating member 32 having the above configuration will be described with reference to FIGS. 14 to 19.

As shown in FIG. 14, a separating member 32A according to a modification is configured such that only the tip end sides of the plurality of comb teeth 34 are tilted. That is, each comb tooth 34 has a bent portion 34c between the base end 34a and the tip end 34b, so that each comb tooth 34 extends linearly in the first direction X from the base end 34a to the bent portion 34c and extends linearly at a tilted angle θ with respect to the third direction Z from the bent portion 34c to the tip end 34b. In this case, the tilt angle θ can be set as appropriate within the range of 30° to 90°.

According to the separating member 32A having the above configuration, the tip end 34b of each comb tooth 34 that is far from the support portion 33 is first inserted into the clearance 23. Thereafter, as each comb tooth 34 is further inserted into the clearance 23, the tilted portion from the tip end 34b to the bent portion 34c acts to correct the deflection in the second direction Y of the separating member 32. The straight portion from the bent portion 34c to the base end 34a is then eventually inserted into the clearance 23. As in the case of the separating member 32, this configuration is also advantageous in that it allows smooth insertion of the comb teeth 34 of the separating member 32A into the clearances 23 between the perch members 22.

As shown in FIG. 15, a separating member 32B according to a modification includes a variable mechanism unit that can change the tilt angle θ of the plurality of comb teeth 34 with respect to the third direction Z. This variable mechanism unit has a function to rotate the support portion 33 about a rotation shaft portion 33a extending in the second direction Y, and in this example, uses an actuator 50. Typically, a motor, an air cylinder, etc. can be used as the actuator 50.

According to the separating member 32B having the above configuration, the tilt angle θ of the plurality of comb teeth 34 can be easily changed by rotating the support portion 33 about the rotation shaft portion 33a.

Although not particularly shown in the figure, the support portion 33 may have a plurality of insertion openings (insertion holes or slits) into which the comb teeth 34 can be inserted at different tilt angles θ. In this case, an insertion opening that allows each comb tooth 34 to be set at a desired tilt angle θ is selected from the plurality of insertion openings. The comb teeth 34 are then inserted into the selected insertion opening. This can simplify the configuration of the variable mechanism structure that can change the tilt angle θ of the plurality of comb teeth 34.

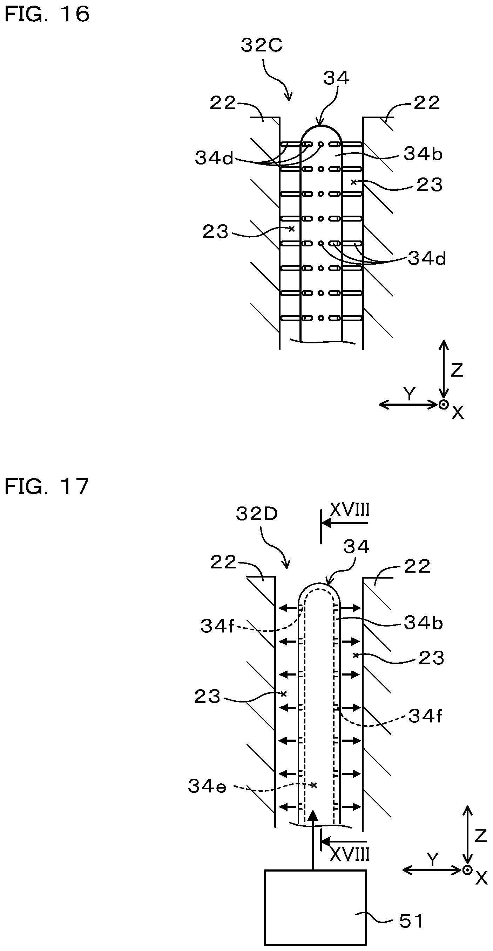

As shown in FIG. 16, in a separating member 32C according to a modification, a flexible intervening portion 34d is provided on the outer periphery of each of the plurality of comb teeth 34 in order to fill the clearance 23. In this example, the intervening portion 34d is a brush composed of a large number of flexible bristles. The bristles of the brush are provided at a plurality of circumferential positions of each comb tooth 34. The material of the bristles of the brush is not particularly limited, and a plant material, an animal material, a metal material, a synthetic fiber material, etc. can be used as appropriate.

According to the separating member 32C having the above configuration, the intervening portion 34d can fill the region in the clearance 23 that cannot be filled only by the comb tooth 34. The organisms C can thus be more effectively scrapped off the clearances 23 between the perch members 22. A flexible fiber material, cloth material, etc. may be used instead of the brush as long as it has the function to fill the clearance 23 like the brush does.

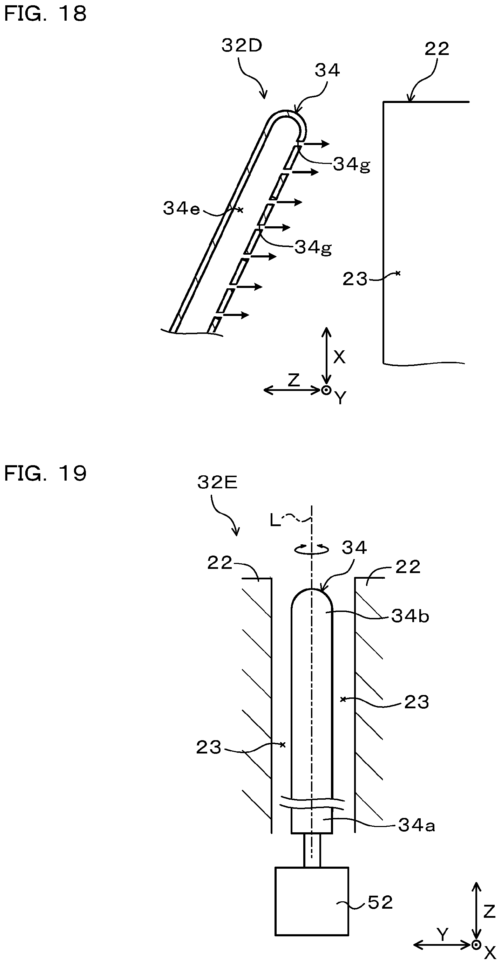

As shown in FIGS. 17 and 18, in a separating member 32D according to a modification, each of the plurality of comb teeth 34 includes a hollow portion 34e, a plurality of first gas injection holes 34f (see FIG. 17) communicating with the hollow portion 34e, and a plurality of second gas injection holes 34g (see FIG. 18) communicating with the hollow portion 34e. By supplying gas from a gas supply source 51 into the hollow portion 34e of each comb tooth 34, the gas is injected to the outside from the gas injection holes 34f and the gas injection holes 34g.

As shown in FIG. 17, when each comb tooth 34 is inserted in the clearance 23, the first gas injection holes 34f are open toward the surfaces of the perch members 22. According to the first gas injection holes 34f, when each comb tooth 34 is inserted in the clearance 23, a strong gas flow is directly applied to the organisms C perching on the surfaces of the perch members 22, which facilitates falling of the organisms C from the perch members 22.

As shown in FIG. 18, the second gas injection holes 34g are open toward the clearance 23 before each comb tooth 34 is inserted into the clearance 23. That is, the second gas injection holes 34g are formed on the front side of each comb tooth 34 in the travel direction relative to the perch members 22. Since the second gas injection holes 34g constantly inject gas toward the organisms C in the clearance 23 while approaching the perch members 22, the living organisms C can be more effectively scraped off.

Air, carbon dioxide gas mainly containing carbon dioxide, nitrogen gas that is an inert gas, etc. can be used as the gas that is injected from the gas injection holes 34f, 34g. When a low oxygen concentration gas such as carbon dioxide gas or nitrogen gas is used, the organisms C can be temporarily put into suspended animation due to deficiency of oxygen. This is effective in easily causing the organisms C to fall from the clearances 23 between the perch members 22.

The gas injected from the gas injection holes 34f, 34g may be a room temperature gas, a low temperature gas whose temperature is lower than the room temperature gas, or a high temperature gas whose temperature is higher than the room temperature gas. When a high temperature gas or a low temperature gas is used, the organisms C in the clearances 23 between the perch member 22 are stimulated, which facilitates escaping of the organisms C from the clearances 23.

As shown in FIG. 19, in a separating member 32E according to a modification, the base end 34a of each comb tooth 34 is connected to an actuator 52. Each comb tooth 34 is rotated on its axis L (that is, rotates) by the driving force of the actuator 52. Typically, a motor, an air cylinder, etc. can be used as the actuator 52.

According to the separating member 32E having the above configuration, the rotating comb teeth 34 are caused to act on the organisms C in the clearances 23 between the perch members 22, which stimulates the organisms C and facilitates escaping of the organisms C from the clearances 23. A mechanism for rotating each comb tooth 34 on its axis L by the driving force of the actuator 52 may be added to at least one of the separating members 32A to 32D described above.

Next, the functions and effects of the first embodiment will be described.

The breeding device 1 of the first embodiment includes the body frame 10, the plurality of breeding units 20, the plurality of perch members 22, and the separating member 32. In this breeding device 1, the plurality of breeding units 20 is housed in the breeding space 11 of the body frame 10. The plurality of perch members 22 arranged with the clearances 23 therebetween is provided in each of the plurality of breeding units 20. The separating member 32 includes the plurality of comb teeth 34 arranged in the second direction Y that is the arrangement direction of the plurality of perch members 22.

The separating member 32 is used for all of the plurality of breeding units 20, and is used to separate the organisms C perching on the plurality of perch members 22 of each breeding unit 20 from the plurality of perch members 22. For this purpose, the separating member 32 is moved relative to each of the plurality of breeding units 20 after the breeding during which the organisms C have grown. The plurality of comb teeth 34 is thus inserted into the plurality of clearances 23 between the plurality of perch members 22 of each breeding unit 20. By causing each comb tooth 34 of the separating member 32 to interfere with the organisms C located in the clearance 23 between the two perch members 22, 22 facing each other, the organisms C can be easily separated from the plurality of perch members 22 of each breeding unit 20. At this time, one separating member 32 need only be moved relative to each of the plurality of breeding units 20. This can reduce the time it takes to separate and collect the organisms C from the plurality of perch members 22 of each breeding unit 20, compared to a structure in which a separating member is prepared for each of the plurality of breeding units 20 and the separate separating members are moved relative to the breeding units 20.

Therefore, according to the first embodiment described above, it is possible to provide the breeding device 1 that is effective in efficiently collecting the bred organisms C in a short time.

Moreover, the use of one separating member 32 for the plurality of breeding units 20 can simplify the structure of the breeding device 1 and reduce the device cost.

In the breeding device 1 of the first embodiment, the plurality of breeding units 20 is arranged side by side in the third direction Z that is the direction in which the separating member 32 and the breeding unit 20 are relatively moved when collecting the organisms C. Therefore, the plurality of breeding units 20 need only be sequentially slid toward the separating member 32 in order from the breeding unit 20 located closer to the separating member 32. This simplifies the work of collecting the organisms C.

In the breeding device 1 of the first embodiment, the body frame 10 includes the plurality of support portions 12a, 12b provided so as to extend in the third direction Z in order to support the plurality of breeding units 20 such that the plurality of breeding units 20 is movable in the third direction Z. This can simplify the structure for moving each of the plurality of breeding units 20 in the moving direction Z1 by the body frame 10.

In the breeding device 1 of the first embodiment, when the collection device 30 is connected to the body frame 10, the plurality of support portions 12a, 12b of the body frame 10 is connected to the plurality of support portions 31a, 31b of the collection device 30. This can simplify the structure for moving each of the plurality of breeding units 20 in the moving direction Z1 from the body frame 10 to the collection device 30 when collecting the organisms C.

Other embodiments related to the first embodiment described above will be described below with reference to the drawings. In other embodiments, the same elements as those of the first embodiment are denoted by the same signs as those of the first embodiment, and description of the same elements will be omitted.

### (Second Embodiment)

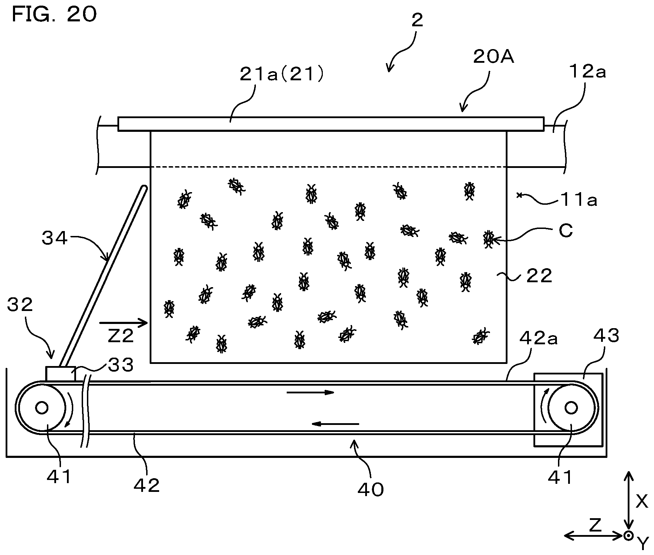

As shown in FIG. 20, a breeding device 2 of a second embodiment is different from the breeding device 1 of the first embodiment in that the separating member 32 is provided in the body frame 10. In this breeding device 2, the first breeding unit 20A and the second breeding unit 20B have the same structure. Therefore, only the first breeding unit 20A is shown in FIG. 20, and description of the second breeding unit 20B will be omitted.

In the breeding device 2, the first breeding unit 20A is fixed to the support portions 12 of the body frame 10. Therefore, although not particularly shown in the figure, the plurality of first breeding units 20A is arranged in a stationary state side by side in the third direction Z that is a horizontal direction.

The separating member 32 is fixed to the conveying surface 42a of the belt 42 of the belt conveyor 40. The conveying surface 42a of the belt 42 is moved under the plurality of first breeding units 20A in a moving direction Z2 by the driving force of the motor 43. Therefore, the belt conveyor 40 is a drive unit that drives the separating member 32 in the moving direction Z2 with respect to each of the plurality of first breeding units 20A.

The other configurations are the same as those of the first embodiment.

After the breeding during which the organisms C have grown, the motor 43 is driven so that the conveying surface 42a of the belt 42 of the belt conveyor 40 moves from left to right in FIG. 20. This allows the separating member 32 to move in the moving direction Z2 toward the plurality of perch members 22 of each first breeding unit 20A. At this time, the plurality of comb teeth 34 of the separating member 32 is inserted into the plurality of clearances 23 between the plurality of perch members 22 of the first breeding unit 20A. The subsequent operations are substantially the same as those described in the first embodiment with reference to FIGS. 10 to 13.

According to the breeding device 2 of the second embodiment, the separating member 32 can be automatically moved in the moving direction Z2 by the belt conveyor 40. Therefore, there is no need for the worker to manually move the breeding unit 20 as in the breeding device 1 of the first embodiment, which can reduce the burden on the worker.

According to the breeding device 2 of the second embodiment, the belt conveyor 40 that is originally for conveyance can be used as a drive unit for moving the separating member 32 in the third direction Z. This is effective in reducing the device cost of the breeding device 2 compared to the case where a dedicated drive unit is provided for the separating member 32. However, a structure in which a dedicated drive unit is provided for the separating member 32 is not excluded, and a drive unit other than the belt conveyor 40 may be used as necessary.

The functions and effects of the second embodiment are otherwise the same as those of the first embodiment.

### (Third Embodiment)

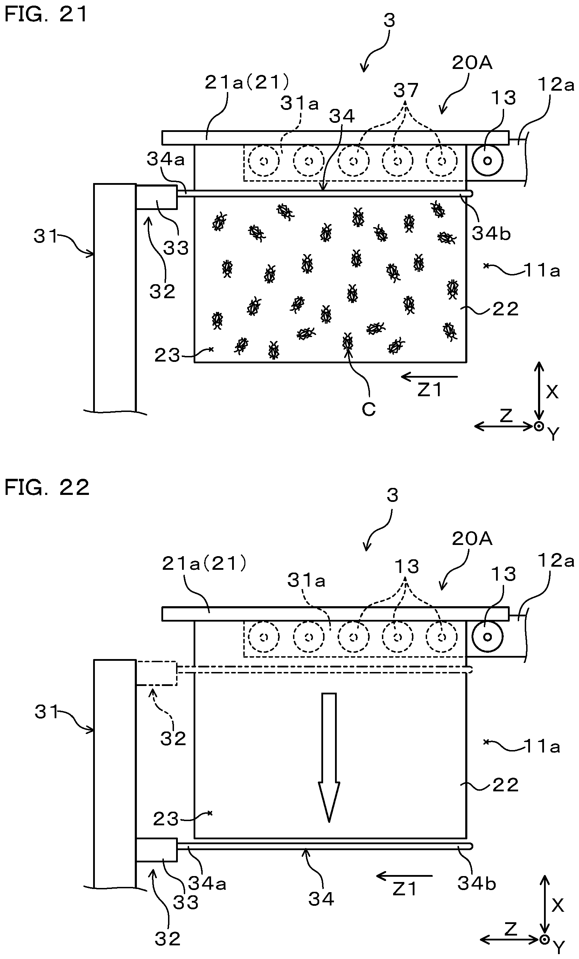

As shown in FIG. 21, a breeding device 3 of a third embodiment is different from the breeding device 1 of the first embodiment in the structure of the separating member 32. In this breeding device 3, the first breeding unit 20A and the second breeding unit 20B have the same structure. Therefore, only the first breeding unit 20A is shown in FIG. 21, and description of the second breeding unit 20B will be omitted.

In the breeding device 3, the separating member 32 is provided on the body portion 31 such that the plurality of comb teeth 34 extending in the third direction Z can be raised and lowered in the first direction X. The separating member 32 may be configured so that the operation of raising and lowering the separating member 32 is performed manually by the worker, or may be configured so that the operation of raising and lowering the separating member 32 is performed automatically using an actuator.

The other configurations are the same as those of the first embodiment.

According to the breeding device 3 of the third embodiment, with the separating member 32 at a raised position, the first breeding unit 20A is slid in the moving direction Z1. At this time, as shown in FIG. 21, with the first breeding unit 20A supported by the support portions 31a, the plurality of comb teeth 34 of the separating member 32 is inserted into the plurality of clearances 23 between the plurality of perch members 22. When the comb teeth 34 are inserted to such an extent that the tip ends 34b protrude from the clearances 23 to the right in FIG. 21, the sliding of the first breeding unit 20A in the moving direction Z1 is stopped.

As shown in FIG. 22, the separating member 32 is lowered from the raised position to a lowered position. The comb teeth 34 of the separating member 32 can thus be caused to interfere with the organisms C in the clearances 23, so that the organisms C can be easily separated from the plurality of perch members 22. Thereafter, with the separating member 32 kept at the lowered position, the first breeding unit 20A is slid again in the moving direction Z1 and passed through the separating member 32. With the separating member 32 raised to the raised position, the remaining first breeding unit 20A standing by in the first breeding space 11a is also slid in the moving direction Z1 in the same manner. The work of collecting the organisms C can thus be sequentially performed for each of the plurality of first breeding units 20A.

The functions and effects of the third embodiment are otherwise the same as those of the first embodiment.

Although the present disclosure is described based on the embodiments, it should be understood that the present disclosure is not limited to such embodiments or structures. The present disclosure includes various modifications and modifications within the equivalent range. In addition, various combinations and forms, and other combinations and forms including only one element, two or more elements, or fewer elements are within the spirit and scope of the present disclosure.

The forms described above illustrates the case where the two breeding spaces 11 are formed in two tiers in the first direction X, namely upper and lower tiers, inside the body frame 10. However, the number of breeding spaces 11 and the arrangement thereof are not limited to this, and can be changed as appropriate when necessary. For example, a structure in which the breeding space 11 is formed in only one tier inside the body frame 10, or a structure in which three or more breeding spaces 11 are formed in a plurality of upper and lower tiers in the first direction X inside the body frame 10 may be used. By increasing the number of tiers of the breeding spaces 11, the upper space in the first direction X can be effectively used, which is effective in increasing the efficiency of breeding the organisms C per installation area of the breeding device.

The forms described above illustrates a structure in which the breeding unit 20 is moved in the moving direction Z1 with respect to the separating member 32 in a stationary state when collecting the organisms C, and a structure in which the separating member 32 is moved in the moving direction Z2 with respect to the breeding unit 20 in a stationary state when collecting the organisms C. However, a structure in which the breeding unit 20 is moved the moving direction Z1 and the separating member 32 is moved in the moving direction Z2 when collecting the organisms C may alternatively be used. The use of the structure in which both the breeding unit 20 and the separating member 32 are moved in a direction toward each other can reduce the time required for the work of collecting the organisms C.

## Claims

1. A breeding device (1, 2, 3) for organisms (C), the breeding device comprising:
a body frame (10) forming a breeding space (11, 11a, 11b) for the organisms;
a plurality of breeding units (20, 20A, 20B) housed in the breeding space of the body frame;
a plurality of perch members (22) arranged in each of the plurality of breeding units with a clearance (23) between the perch members; and
a separating member (32) including a plurality of comb teeth (34) arranged in an arrangement direction (Y) of the plurality of perch members, wherein the separating member is used for all of the plurality of breeding units, and is configured in such a manner that the plurality of comb teeth is inserted into a plurality of the clearances between the plurality of perch members by moving the separating member relative to each of the plurality of breeding units.

2. The breeding device according to claim 1, wherein
the plurality of breeding units is arranged side by side in a horizontal direction (Z), and
the separating member moves relative to each of the plurality of breeding units in the horizontal direction.

3. The breeding device according to claim 2, wherein the body frame includes a support portion (12a, 12b) for supporting the plurality of breeding units in such a manner that the plurality of breeding units is movable with respect to the separating member in a stationary state in the horizontal direction.

4. The breeding device according to claim 3, wherein
the separating member is provided in a collection device (30) separate from the body frame, and
the collection device includes a second support portion (31a, 31b) for supporting the plurality of breeding units in such a manner that the plurality of breeding units is movable in the horizontal direction, when connected to the body frame, the collection device guides the plurality of breeding units in such a manner that the plurality of breeding units is continuously movable from a first support portion (12a, 12b) to the second support portion, and with each of the plurality of breeding units supported by the second support portion, the plurality of comb teeth is inserted into the plurality of clearances, the first support portion being the support portion of the body frame.

5. The breeding device according to claim 1, wherein
the plurality of breeding units is arranged side by side in a horizontal direction (Z) in a stationary state, the breeding device further comprising
a drive unit (40) that drives the separating member with respect to each of the plurality of breeding units in the horizontal direction.

6. The breeding device according to claim 5, wherein the drive unit is a belt conveyor (40) including a belt (42) that moves in the horizontal direction under the plurality of breeding units, and the separating member is fixed to the belt of the belt conveyor.

7. The breeding device according to any one of claims 1 to 6, wherein
each of the plurality of breeding units includes a holding portion that holds the plurality of perch members, and the plurality of perch members extends such that base ends (22a) of the plurality of perch members are ends located closer to the holding portion, and
the separating member includes a support portion (33) that supports the plurality of comb teeth, the plurality of comb teeth extends from the support portion to tip ends (34b) and is configured such that the tip ends of the plurality of comb teeth are inserted into the plurality of clearances at the base ends of the plurality of perch members.

8. The breeding device according to claim 7, wherein the plurality of comb teeth of the separating member is tilted at a constant tilt angle (θ) from the support portion to the tip ends with respect to a direction of movement relative to the plurality of breeding units.

9. The breeding device according to claim 8, wherein the separating member includes a variable mechanism unit (50) configured to change the tilt angle of the plurality of comb teeth.

10. The breeding device according to any one of claims 1 to 9, wherein each of the plurality of comb teeth has a plurality of gas injection holes (34f, 34g), and gas is injected from the gas injection holes to outside.

11. The breeding device according to claim 10, wherein the plurality of gas injection holes includes a first gas injection hole (34f) that is open toward a surface of the perch member when the comb tooth is inserted in the clearance.

12. The breeding device according to claim 11, wherein the plurality of gas injection holes includes a second gas injection hole (34g) that is open toward the clearance before the comb tooth is inserted into the clearance.

13. The breeding device according to any one of claims 1 to 12, wherein a flexible intervening portion (34d) is provided on an outer periphery of each of the plurality of comb teeth in order to fill the clearance.
